(19) Europäisches Patentamt
European Patent Office
Office européen des brevets

(11) Veröffentlichungsnummer: **0 085 881**
**A2**

(12) # EUROPÄISCHE PATENTANMELDUNG

(21) Anmeldenummer: 83100611.9

(22) Anmeldetag: 25.01.83

(51) Int. Cl.³: C 07 D 498/04
C 07 D 513/04, C 07 D 471/04
C 07 D 498/14, A 61 K 31/535
A 61 K 31/54, A 61 K 31/47
//(C07D498/04, 265/00, 231/00), (C07D513/04, 279/00, 231/00), (C07D471/04, 231/00, 221/00), (C07D498/14, 265/00, 231/00, 221/00), (C07D498/14, 317/00, 265/00, 231/00)

(30) Priorität: 06.02.82 DE 3204126

(43) Veröffentlichungstag der Anmeldung: 17.08.83 Patentblatt 83/33

(84) Benannte Vertragsstaaten: AT BE CH DE FR GB IT LI LU NL SE

(71) Anmelder: BAYER AG
Zentralbereich Patente, Marken und Lizenzen
D-5090 Leverkusen 1, Bayerwerk(DE)

(72) Erfinder: Mardin, Mithat, Dr.
Untere Bergerheide 15
D-5600 Wuppertal 1(DE)

(72) Erfinder: Sundermann, Rudolf, Dr.
Treptower Strasse 5
D-5090 Leverkusen(DE)

(72) Erfinder: Hoffmeister, Friedrich, Prof. Dr.
Katernberger Strasse 262
D-5600 Wuppertal 1(DE)

(72) Erfinder: Busse, Wolf-Dieter, Dr.
Pahlkestrasse 65
D-5600 Wuppertal 1(DE)

(72) Erfinder: Horstmann, Harald, Dr.
Claudiusweg 19
D-5600 Wuppertal 1(DE)

(72) Erfinder: Raddatz, Siegfried, Dr.
Jakob Böhme Strasse 21
D-5000 Köln 80(DE)

(54) **Pyrazolooxazine, -thiazine, -chinoline, Verfahren zu ihrer Herstellung und ihre Verwendung als Arzneimittel.**

(57) Die vorliegende Erfindung betrifft Pyrazolooxazine, -thiazine, -chinoline, ein Verfahren zu ihrer Herstellung, ihre Verwendung als Arzneimittel, insbesondere ihre Verwendung als Lipoxygenasehemmer, diese enthaltende Arzneimittel und deren Herstellung.

EP 0 085 881 A2

0085881

BAYER AKTIENGESELLSCHAFT 5090 Leverkusen, Bayerwerk

Zentralbereich
Patente, Marken und Lizenzen Je/Kü

# Pyrazolooxazine, -thiazine, -chinoline, Verfahren zu ihrer Herstellung und ihre Verwendung als Arzneimittel

Die vorliegende Erfindung betrifft Pyrazolooxazine, -thiazine, -chinoline, ein Verfahren zu ihrer Herstellung, ihre Verwendung als Arzneimittel, insbesondere ihre Verwendung als Lipoxygenasehemmer, diese enthaltende Arzneimittel und deren Herstellung.

Es ist bekannt, daß die durch das Enzym Lipoxygenase gebildeten Metaboliten der Arachidonsäure-Leukotriene und slow reacting Substance of anaphylaxis (SRS-A) - an der Entstehung von entzündlichen und allergischen Prozessen beteiligt sind, vgl. E.J. Goetzl, Immunology 40 709 (1980) und Medical Clinis of North America 65 , 809 (1981); Samuelsson et. al., Trends in Pharmacol. Sci. Mai 1980, 227 und Blood, 58, 658 (1981); Borgeat et. al., J. Med. Chem. 24, 121 (1981) und Int. J. Immunopharmac. 2, 281-293, (1980); Austen und Lewis, Nature, 293, 103 (1981).

Bekannte Lipoxygenasehemmer wie Nordihydroguaretsäure, 3-Amino-1-(3-trifluormethylphenyl)-pyrazolin, Phenidon und 5,8,11,14-Eikosatetrainsäure sind entweder gleichzeitig

als Cyclooxygenasehemmer oder erst bei sehr hohen Konzentrationen wirksam. Die Hemmung des Enzyms Cyclooxygenase des Arachidonsäuremetabolismus führt zu einer globalen Prostaglandinsynthesehemmung und zu einer Stimulation des Lipoxygenaseweges, was eine Gastrotoxizität bzw. pro-inflammatrische und asthmatische Wirkungen verursacht (vgl. Yen und Kreutner, Agents and Actions, 10, 274 (1980) und Blackwell und Flower, Prostaglandins 16, 417 (1978); und vgl. ebenso Brune et al., J. Pharm. Pharmacol. 33, 127 - 128 (1981); Higgs et al., Eur. J. Pharmacol. 66, 81 - 86 (1980) und Piper et al., Prostaglandins 19, 371 (1980)). Außerdem haben schon bekannte Lipoxygenasehemmer wie 3-Amino-1-(m-trifluormethylphenyl) pyrazolin -2 bei systemischer Verabreichung (z.B. oral) toxische Nebenwirkungen. Es besteht deshalb ein Bedarf nach oral wirksamen Verbindungen, die diese unerwünschten Nebenwirkungen nicht besitzen.

Überraschenderweise hemmen die erfindungsgemäßen Pyrazolderivate die Lipoxygenase bereits in solcher Konzentrationen, bei denen die Cyclooxygenase wenig beeinflußt wird.

Die erfindungsgemäßen Verbindungen stimulieren überraschenderweise auch die Synthese von Prostacyclin in arteriellen Gefäßen in vitro, möglicherweise als Folge ihrer lipoxygenasehemmenden Eigenschaft. (Gryglewski et al. Prostaglandins 89, 685 (1976)). Bezüglich dieser Wirkung sind die Verbindungen stärker wirksam als der genannte Lipoxygenasehemmer 3-Amino-1-(m-trifluormethylphenyl)-pyrazolin-2. (Proc. of British Pharmacological Society 920 P (1981). Die erfindungsgemäßen

Verbindungen stimulieren auch an Kaninchenaortastreifen die Prostacyclin-Synthese.

Die erfindungsgemäßen Verbindungen besitzen auch eine antiphlogistische Wirkung im Carrageenan-induzierten Ödemmodell, wenn sie systemisch, besonders oral, und lokal, besonders cutan, verabreicht werden. Sie besitzen ebenfalls antimetastatische Wirkung.

Die erfindungsgemäßen, lipoxygenasehemmenden Pyrazoloderivate sind somit als Arzneimittel bei der Behandlung von entzündlichen und allergischen Prozessen und als Analgetika einsetzbar. Sie können insbesondere als Antiphlogistika, Antirheumatika, Antiatherosklerotika, Antithrombotika, Antiartroika, Antiasthmatika, Antiallergika, Antimetastatika, Antihypertensiva und Gastroprotektiva Verwendung finden.

Die erfindungsgemäßen Pyrazolooxazine, -thiazine und -chinoline entsprechen der allgemeinen Formel I in ihren isomeren Formen Ia und Ib

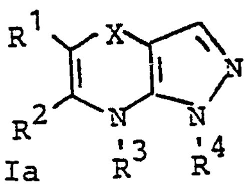
Ia

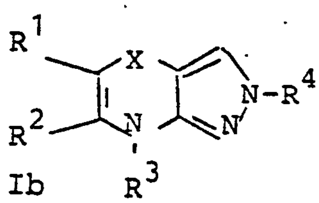
Ib

(I)

worin

X Sauerstoff, Schwefel, SO, $SO_2$, Methylen ($CH_2$) bedeutet,

$R^1$ und $R^2$ für einen annellierten Aryl- oder Heteroarylring stehen, der gegebenenfalls seinerseits mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino, substituiertes Amino, Hydroxy, Sulfonamido, Methylencarboxy, Methylencarboxylalkyl, Propylencarboxyalkyl oder eine Dioxymethylengruppierung substituiert sein kann,

$R^3$ und $R^4$ gleich oder verschieden sein können und für Wasserstoff, Alkyl mit 1 bis 18 C-Atomen wobei der Alkylrest durch Sauerstoff, Schwefel oder Stickstoff ein- oder mehrfach unterbrochen sein kann, Alkenyl oder Alkinyl mit 2 bis 12 C-Atomen, Aralkyl mit 7 bis 12 C-Atomen, wobei die Alkenyl-, Alkinyl- und Aralkylreste ebenfalls durch Sauerstoff, Schwefel oder Stickstoff ein- oder mehrmals unterbrochen sein können, Aryl mit 6 bis 10 C-Atomen, Heteroaryl, Heteroaralkyl, Alkylcarbonyl mit 1 bis 18 C-Atomen, Heteroarylcarbonyl und Arylcarbonyl mit 7 bis 11 C-Atomen stehen, wobei diese Reste gegebenenfalls mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino- oder substituiertes Amino, Hydroxy, Sulfonamido, Methylencarboxy, Methylencarboxyalkyl oder Propylencarboxyalkyl substituiert sein können.

Wenn nachfolgend Bezug auf die allgemeine Formel I genommen wird, so sind immer beide isomere Formen vorstehend mit Ia und Ib gekennzeichnet, gemeint. Bevorzugt sind Pyrazolooxazine, -thiazine und -chinoline der allgemeinen Formel I, in welcher

$R^1$ und $R^2$ für einen anellierten Aryl- oder Heteroarylring stehen, der seinerseits wie oben aufgeführt substituiert sein kann,

$R^3$ und $R^4$ gleich oder unterschiedlich sein können und für Wasserstoff, Alkyl mit 1 bis 18 C-Atomen, wobei der Alkylrest durch Sauerstoff, Schwefel oder Stickstoff ein- oder mehrmals unterbrochen sein kann, Aralkyl mit 7 bis 12 C-Atomen, Heteroaralkyl,, wobei die Aralkyl- und Heteroaralkylreste durch Sauerstoff, Schwefel und Stickstoff ein- oder mehrmals unterbrochen sein können, Aryl mit 6 bis 10 C-Atomen und Heteroaralkyl stehen,

und X die oben angegebene Bedeutung hat.

Besonders bevorzugt sind erfindungsgemäße Verbindungen der allgemeinen Formel I, worin

$R^1$ und $R^2$ für einen anellierten Benzo- oder Pyridoring stehen, der seinerseits wie oben aufgeführt, substituiert sein kann,

$R^3$ und $R^4$ ungleich sind, wobei $R^3$ oder $R^4$ Wasserstoff

sein kann, während der andere Rest Alkyl mit 1 bis 18 C-Atomen, Aralkyl mit 7 bis 12 C-Atomen, Heteroaralkyl bedeutet, wobei die Alkyl-, Aralkyl- und Heteroaralkylreste durch Sauerstoff, Schwefel und Stickstoff ein- oder mehrmals unterbrochen sein können

und X für Sauerstoff, Schwefel oder Methylen steht.

Ferner fallen unter die vorliegende Erfindung neue Verbindungen der allgemeinen Formel I, wobei auch hier jeweils die beiden isomeren Formen Ia und Ib gemeint sind, sowie ihre Verwendung auch in Arzneimitteln und bei der Bekämpfung von Krankheiten, bevorzugt von entzündlichen Prozessen insbesondere als Lipoxygenasehemmer sowie als die Prostacyclin-Synthese stimulierende Substanzen und als Gefäßwand- und/oder Cytoprotektiva, insbesondere als Antithrombotikum, Antimetastatikum, Antiatherosklerotikum und/oder Ulcus-Präventivum.

Diese neuen Verbindungen sind Verbindungen der allgemeinen Formel I in ihren isomeren Formen

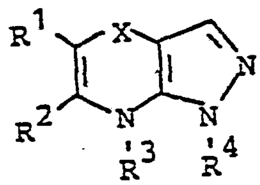

(I)

worin

X Sauerstoff, Schwefel, SO, $SO_2$, Methylen ($CH_2$) bedeutet,

$R^1$ und $R^2$ für einen anelierten Aryl- oder Heteroarylring stehen, der gegebenenfalls seinerseits mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino, substituiertes Amino, Hydroxy, Sulfonamido, Methylencarboxy, Methylencarboxyalkyl, Propylencarboxyalkyl oder eine Dioxymethylengruppierung substituiert sein kann.

$R^3$ und $R^4$ gleich oder verschieden sein können und für Wasserstoff, Alkyl mit 1 bis 18 C-Atomen wobei der Alkylrest durch Sauerstoff, Schwefel oder Stickstoff ein- oder mehrfach unterbrochen sein kann, Alkenyl oder Alkinyl mit 1 bis 12 C-Atomen, Aralkyl mit 7 bis 12 C-Atomen, wobei die Alkenyl-, Alkinyl- und Aralkylreste ebenfalls durch Sauerstoff, Schwefel oder Stickstoff ein- oder mehrmals unterbrochen sein können, Aryl mit 6 bis 10 C-Atomen, Heteroaryl, Heteroaralkyl, Alkylcarbonyl mit 1 bis 18 C-Atomen, Heteroarylcarbonyl und Arylcarbonyl mit 7 bis 11 C-Atomen stehen, wobei diese Reste gegebenenfalls mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy,

Halogen, Amino- oder substituiertes Amino, Hydroxy, Sulfonamido, Methylencarboxy, Methylencarboxyalkyl oder Propylencarboxyalkyl substituiert sein können jedoch mit der Einschränkung:

$R^1$ und $R^2$ bilden zusammen einen Benzoring
und für X = Sauerstoff bedeuten
$R^3$ = Wasserstoff und/oder Methyl und
$R^4$ = Wasserstoff und/oder Methyl
und für X = Schwefel bedeuten
$R^3$ = Wasserstoff und $R^4$ = p-Chlorphenyl oder
$R^3$ = Acyl und $R^4$ = Aryl.

Die erfindungsgemäßen Verbindungen der allgemeinen Formel I lassen sich herstellen, indem man Oxazinone, Thiazinone und Piperidinone der allgemeinen Formel II,

II

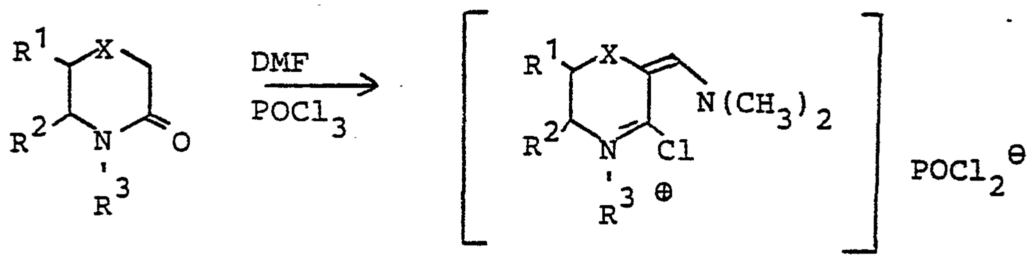


III

in welcher $R^1$, $R^2$, $R^3$ und X die oben angegebene Bedeutung haben mit Dimethylformamid und Phosphoroxychlorid im Sinne einer Vilsmeier-Haack-Reaktion umsetzt (vergl. Mazharuddin und Thagarajan, Tetrahedron Letters 307, 1971) und entweder die dabei entstehenden Salze der allgemeinen Formel III oder die daraus nach Hydrolyse entstehenden Aldehyde III a,

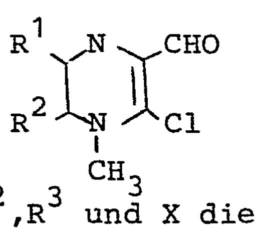 . III a

in denen $R^1$,$R^2$,$R^3$ und X die oben angegebene Bedeutung haben,

mit Hydrazinen der allgemeinen Formel IV

$$R^5 - NH - NH_2 \qquad IV$$

umsetzt, worin $R^5$ Wassertoff, Alkyl mit 1 bis 18 C-Atomen, Aralkyl mit 7 bis 12 C-Atomen, Heteroaralkyl, wobei die Alkyl-, Aralkyl- und Heteroaralkylreste durch Sauerstoff, Schwefel und Stickstoff ein- oder mehrmals unterbrochen sein können und wobei Alkyl z.B. durch Alkoxycarbonyl substituiert sein kann, Aryl mit 6 bis 10 C-Atomen und Heteroaryl bedeutet, wobei die Aryl- und Heteroarylreste mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Halogenalkyl, Halogenalkoxy, Halogen, Amino oder substituiertes Amino, Hydroxy, Sulfonamido, Methylencarboxy, Methylencarboxyalkyl oder Propylencarboxyalkyl substituiert sein können.

Die Umsetzung der Salze der allgemeinen Formel III mit den Hydrazinen der allgemeinen Formel IV, geschieht zweckmäßigerweise in polaren Lösungsmitteln, z.B. in Alkanolen, ohne Katalysator oder auch in Gegenwart von organischen Basen wie z.B. Triethylamin bei Temperaturen zwischen -80°C und 100°C.

Bevorzugt setzt man die Vilsmeier-Haack-Salze der allgemeinen Formel III oder die daraus erhaltenen Aldehyde III a mit den Hydrazinen der allgemeinen Formel IV in Ethanol bei 80°C um, um die erfindungsgemäßen Verbindungen zu erhalten. (Verg. Aki und Nakagawa, Chem. Pharm. Bull. 20, 1325, 1972).

Die erfindungsgemäßen Verbindungen der allgemeinen Formel I, in der $R^3$ und $R^4$ für Alkylcarbonyl und/oder Arylcarbonyl mit der oben angegebenen Bedeutung stehen, lassen sich herstellen, indem man die erfindungsgemäßen Verbindungen der allgemeinen Formel I, in der $R^3$ und/oder $R^4$ für Wasserstoff stehen, mit Acylierungsmitteln, z.B. Säurehalogeniden oder Anhydriden der allgemeinen Formel V, in der $R^5$ die gleiche Bedeutung wie $R^3$ und $R^4$ hat mit der Einschränkung, daß $R^5$ nicht für Wasserstoff steht, umsetzt.

$$(V) \quad R^5-\overset{\overset{O}{\|}}{C}-Y \qquad (Y = \text{Imidazol, } OCOR^5\text{, Hal})$$
$$(\text{Hal} = Cl,\ Br,\ \text{Jod})$$

Die für die Durchführung der Erfindung geeigneten Verbindungen der Formel II sind bekannt oder lassen sich nach bekannten Methoden herstellen. (Vgl. hierzu Shah et al, Indian Journal of Chemistry 7, 1006 (1969) und 10, 820 (1972)).

Die für die Durchführung der Erfindung geeigneten Hydrazine der allgemeinen Formel IV sind bekannt oder lassen sich nach bekannten Methoden herstellen.

Beispiele erfindungsgemäßer Verbindungen sind Pyrazolo[4,3-b][1,4] benzoxazin
7-Methyl-pyrazolo[4,3-b][1,4]benzoxazin
7-Chlor- " " " "
5,7-Dichlor-" " " "
6-Dimethylsulfonamido-pyrazolo[4,3-b][1,4]benzoxazin
1-Methyl- " " " "
1-Methyl-7-chlor- " " " "

1-Hydroxyethyl- " " " "
1-Phenyl- " " " "
9-Methyl- " " " "
9-Ethyl- " " " "
Pyrazolo[4,3-b][1,4]benzothiazin
7-Chlor- " " "
7-Methyl- " " "
1-Phenyl-7-methyl-Pyrazolo[4,3-b][1,4]benzothiazin
4-Dioxo- " " " "
Pyrazolo[4,3-b][1,4]pyrido[3,2-b]oxazin
Pyrazolo[4,3-b][1,4]naphthoxazin
1-Methyl-Pyrazolo[4,3-b][1,4]naphthoxazin
1-Phenyl " " " "

Der Nachweis der lipoxygenasehemmenden Eigenschaften der erfindungsgemäßen Verbindungen, erfolgt analog der Methode von Bailey et al., Journal of Biol. Chemistry 255, 5996, (1980) und nach Blackwell und Flower, Prostaglandins 16, 417 (1978). Bei dieser Testmethode wird der Metabolismus radioaktiv markierter Arachidonsäure an gewaschenen Humanthrombozyten herangezogen. Bei diesem in vitro Test werden die radioaktiv markierten Metaboliten aus dem Reaktionsansatz extrahiert und dünnschichtchromatographisch getrennt. Das Autoradiogramm wird am Dünnschicht-Scanner ausgewertet. Bei diesen Testbedingungen werden die markierten Metaboliten von der nicht umgesetzten Arachidonsäure getrennt und sind anschließend quantitativ auswertbar. Die Verteilung der Radioaktivität auf die während der Metabolisierung gebildeten Cyclooxygenaseprodukte Thromboxan $B_2$ ($TXB_2$) und 12-Hydroxy-5,8,

10-heptadekatriensäure (HHT) bzw. das Lipoxygenaseprodukt 12-Hydroxy-5,8,11,14-eikosatetraensäure (HETE) unter dem Einfluß der Inhibitoren stellt ein Maß für die Hemmung der Enzyme dar.

Die Lipoxygenasehemmung der erfindungsgemäßen Verbindungen läßt sich an der Hemmung der HETE-Synthese messen. Es zeigt sich, daß die Synthese von $TXB_2$ und von HHT unbeeinflußt bleibt, während der Arachidonsäureumsatz abnimmt. Wie aus der nachfolgenden Tabelle ersichtlich ist, bewirken die erfindungsgemäßen Verbindungen eine signifikante Hemmung der Plättchenlipoxygenase (HETE-Synthese). (Vergl. Tab. 1)

Analog dem o.a. Test lassen sich die lipoxygenasehemmenden Eigenschaften der erfindungsgemäßen Verbindungen auch an Leukozyten nachweisen.

Die polymorphkernigen Leukozyten des Menschen und des Kaninchens metabolisieren die Arachidonsäure zu 5-Hydroxy-5,8,11,14-eikosatetraensäure (5-HETE) und Leukotrien $B_4$ (5S, 12R-Dihydroxy-6 cis, 8,10-trans-14 ciseikosatetraensäure). Die Hemmung der Freisetzung von 5-HETE und Leukotrien $B_4$ aus den Leukozyten stellt ein Maß für den lipoxygenasehemmenden Effekt der erfindungsgemäßen Verbindungen dar. (Vergl. Tab. 1)

Der Test mit den Humanleukozyten wird nach Borgeat und Samuelsson (j. Biol. Chem. 254; 2643, 1979 und Proc. Natl. Acad. Sci. USA, 76, 2148 1979), mit Kaninchenleukozyten nach Walker und Parish (Inter. Archs. Allergy appl. Immun. 66, 83, 1981) durchgeführt.

Der Nachweis der Prostacyclin-stimulierenden Wirkung erfolgte durch Bestimmung der Freisetzung von Prostacyclin nach einstündiger Inkubation von Kaninchenaortenstreifen mit den erfindungsgemäßen Verbindungen (analog nach Moncada et al., Lancet 1977, I, 18) und anschließender radioimmunologischer Bestimmung des stabilen Prostacyclinmetaboliten 6-Keto-PGF 1 (B.M. Peskar et al. FEBS Letters 121, 25, 1980) (vgl. Tab. 2).

Die erfindungsgemäßen Verbindungen sind auch in vivo wirksam. Diese Wirkung wird durch die Messung der Hemmung der Leukozytenmigration nach an sich bekannten Methoden nachgewiesen (vgl. Higgs et al., Biochemical Pharmacology 28, 1959, (1979) und Eur. J. Pharmacol. 66, 81 (1981)). Die erfindungsgemäßen Verbindungen reduzieren auch die Ödembildung im Carrageenan-Entzündungsmodell, (vgl. Tab. 3) und hemmen die Metastasierung von B-16 Melanom (nach Honn, et.al. Science 212, 1270, 1981)..

Die neuen Wirkstoffe können in bekannter Weise in die üblichen Formulierungen wie Tabletten, Kapseln, Dragees, Pillen, Granulate, Aerosole, Sirupe, Emulsionen, Suspensionen und Lösungen, unter Verwendung inerter, nichttoxischer pharmazeutisch geeigneter Trägerstoffe oder Lösungsmittel überführt werden. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung

0085881

von Emulgiermitteln und/oder Dispergiermitteln, wobei z.B. im Falle der Benutzung von Wasser als Verdünnungmittel gegebenenfalls organische Lösungsmittel als Hilfslösungsmittel verwendet werden können.

Als Hilfsstoffe seien beispielhaft aufgeführt:

Wasser, nicht-toxische organische Lösungsmittel, wie Paraffine (z.B. Erdölfraktionen), pflanzliche Öle, (z.B. Erdnuß-/Sesamöl), Alkohole (z.B. Ethylalkohol, Glycerin), Glykole (z.B. Propylenglykol, Polyethylenglykol), N-Alkylpyrrolidone, feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel, wie nicht-ionogene anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere cutan und in Aerosolform. Im Falle der oralen Anwendung können Tabletten selbstverständlich außer den genannten Trägerstoffen auch Zusätze wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie

Magnesiumstearat, Natriumlaurylsulfat und Talkum zum Tablettieren mitverwendet werden. Im Falle wäßriger Suspensionen und/oder Elixieren, die für orale Anwendungen gedacht sind, können die Wirkstoffe außer mit den obengenannten Hilfsstoffen mit verschiedenen Geschmacksaufbesserern oder gefärbten bzw. farbgebenden Stoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,05 bis 5 mg/kg Körpergewicht pro Tag zur Erzielung wirksamer Ergebnisse zu verabreichen, und bei oraler Applikation beträgt die Dosierung etwa 0,05 bis 100 mg/kg, vorzugsweise 0,1 bis 10 mg/kg Körpergewicht pro Tag.

Trotzdem kann es gegebenenfalls erforderlich sein, von den genannten Mengen abzuweichen, und zwar in Abhängigkeit vom Körpergewicht bzw. der Art des Applikationsweges, aber auch aufgrund der Art und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle

der Applikation größerer Mengen kann es empfehlenswert sein, diese in mehrere Einzelgaben über den Tag zu verteilen. Die obigen Ausführungen gelten für die Applikation sowohl in der Human- als auch in der Tiermedizin in sinngemäß gleicher Weise.

Die folgenden Beispiele sollen die Erfindung näher erläutern: (siehe auch Tabelle 1).

<u>Beispiel 1</u> (Verb. Nr. 1 aus Tab 1)

14,9 g (0,1 Mol) Benzoxazinon werden in 50 ml absolutem DMF gelöst. Bei Raumtemperatur läßt man 18,4 g (0,12 Mol) $POCl_3$ langsam zutropfen, dabei steigt die Temperatur bis 80°C an. Nach beendeter Reaktion kristallisiert beim Abkühlen ein rotes Salz aus, das abgesaugt und in Alkohol gelöst wird. Zu dieser Lösung werden langsam 12 g (0,24 Mol) Hydrazinhydrat zugetropft. (Selbstwärmung, Dimethylamin-Entwicklung). Nach beendeter Reaktion wird eine halbe Stunde unter Rückfluß gekocht und anschließend mit Wasser verdünnt. Beim Abkühlen kristallisiert das Produkt aus.

Fp. 220°C Zers. (aus verd. Alkohol)
Ausbeute: 15 g (87 %).

Herstellung des Benzoxazinons ( Formel II, $R_1$ und $R_2$= Benzo; $R_3$=H)

In einem 1 Ltr.-Erlenmeyerkolben werden 109 g (1 Mol) o-Aminophenol in 500 ml Wasser mit 85 ml konz. Salzsäure gelöst und 5 Minuten mit 15 g A-Kohle gerührt. Nach dem Absaugen der A-Kohle muß das Filtrat wasserklar sein, dann wird mit Natronlauge vorsichtig auf pH 6 - 7 gestellt. Das dabei ausfallende Produkt wird abgesaugt und mit Wasser gewaschen.

Das so gereinigte, noch feuchte o-Aminophenol wird in 350 ml Wasser und 310 ml Aceton gelöst. Zu dieser Lösung werden unter Rühren gleichzeitig 113 g (1 Mol)

frisch destilliertes Chloracetylchlorid und aus einem zweiten Tropftrichter Natronlauge zugetropft, so daß ein pH-Wert bei 3 bis 4 gehalten wird. Nach beendeter Reaktion wird mit Natronlauge auf pH 7 gestellt und das Aceton abdestilliert. Nun wird mit konz. Natronlauge bei 70°C stark alkalisch gestellt und 10 Minuten unter Rückfluß gekocht. Nach dem Abkühlen auf 30°C wird nun mit konz. Salzsäure auf pH 1 gestellt und kurz aufgekocht. Nach dem Abkühlen wird das ausgefallene Produkt abgesaugt, mit Wasser gewaschen und getrocknet.

Ausbeute: 98 g, Fp. 173°C.

Beispiel 2 (Verb. Nr. 3, Tab. 1)

In 300 ml wasserfreies Dimethylformamid werden unter Eiskühlung langsam 50 g (0,24 Mol) $PCl_5$ eingetragen. Bei 20°C wird nun eine Lösung von 48 g (0,22 Mol) Dichlorbenzmorpholon in 100 ml Dimethylformamid schnell zugegebe. Dabei steigt die Temperatur bis auf ca. 50°C an. anschließend wird 1 Stunde bei 80°C nachgerührt. Nach Abkühlen auf Raumtemperatur werden die roten Kristalle abgesaugt und mit wenig Dimethylformamid gewaschen.

Fp. 202°C.
Ausbeute: 46 g (64 % der Theorie).

$PCl_5$ ist bei dieser Reakion unbedingt erforderlich, da sonst (z.B. mit $POCl_3$) die Lactamgruppe nicht vollständig in das Lactimchlorid überführt wird.

Zu einer Lösung von 15 ml Hydrazinhydrat in 70 ml Ethanol werden 11,5 g des oben hergestellten Salzes eingetragen und unter Rückfluß gekocht. Nach beendeter Reaktion werden die ausgefallenen hellgelben Kristalle abgesaugt und getrocknet.

Fp. 230°C
Ausbeute: 6,4 g (76 %).

Beispiel 3 (Verb. Nr. 45, Tab. 1)

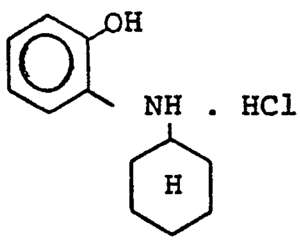

2-Cyclohexylaminophenol-Hydrochlorid

Eine Mischung von 10,9 g (0,1 Mol) 2-Aminophenol und 10,4 g (0,1 Mol)Cyclohexanon in 200 ml Ethanol wird unter Normalbedingungen über 0,2 g $PtO_2$ hydriert, das Reaktionsgemisch mit 0,1 Mol HCl in Isopropanol versetzt, der Katalysator abfiltriert, das Filtrat i. Vak. eingedampft und der Rückstand mit Ether ausgerührt.

Ausbeute: 16 g (70,3 % d.Th.), Schmp. 258-60°C.

Beispiel 4

4-Cyclohexyl-benzoxazin/1,4/-on-3

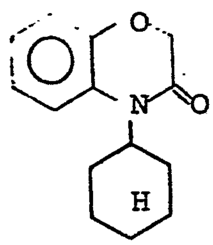

Eine Suspension von 4,55 g (20 mMol) 2-Cyclohexylaminophenol-hydrochlorid in 60 ml abs. Toluol wird mit 2,77 ml (20 mMol) Triethylamin und anschließend mit 1,6 ml (20 mMol) Chloracetylchlorid versetzt und 1 Std. unter Rückfluß erhitzt. Die Suspension wird mit Wasser ausgeschüttelt, die organische Phase nach Behandeln mit Aktivkohle über $Na_2SO_4$ getrocknet und abdestilliert. Das verbleibende Öl wird in 100 ml Ethanol aufgenommen und nach Zusatz von 3,1 g Kaliumacetat 1 Std. unter Rückfluß erhitzt. Der nach Verdampfen des Lösungsmittels verbleibende Rückstand wird an Kieselgel mit Essigester/Dichlormethan (1:4) chromatographiert.

Ausbeute: 2,1 g (69 % d.Th.), Öl.

Beispiel 5

9-Cyclohexyl-pyrazolo/4,3-b/1,4/benzoxazin

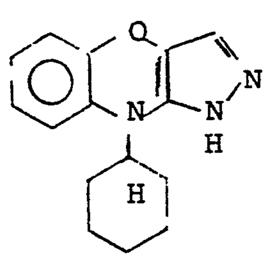

Eine Lösung von 55 ml (0,716 Mol) DMF in 55 ml Ethylenchlorid wird unterhalb 0°C mit 55 ml (0,598 Mol) $POCl_3$ versetzt. Man erwärmt die Suspension auf Raumtemperatur, fügt eine Lösung von 53,6 g (0,232 Mol) 4-Cyclohexylbenzoxazin-[1,4]-on-3 hinzu, kocht 1 Std. unter Rückfluß und destilliert das Lösungsmittel ab. Der Rückstand wird in 600 ml Ethanol aufgenommen, unterhalb 30° mit 83 ml (1,7 Mol) Hydrazinhydrat versetzt und das Gemisch über Nacht bei Raumtemperatur aufbewahrt. Das Lösungsmittel wird verdampft, der Rückstand zwischen Wasser und Dichlormethan verteilt, die organische Phase über $Na_2SO_4$ getrocknet, eingedampft und der Trockenrückstand an Kieselgel mit Essigester/Dichlormethan chromatographiert.
Ausbeute 21,2 g (36 % d.Th.) Schmp. 134-35°C (aus Ether/Petrolether).

Beispiel 6 (Verb. Nr. 39, Tab. 1)

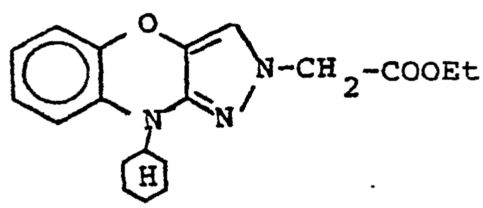

Ethyl-[9-cyclohexyl-pyrazolo[4,3-b][1,4]benzoxazin-2-yl]-acetat

Eine Lösung von 5,1 g (20 mMol) 9-Cyclohexylpyrazolo-[4,3-b][1,4]benzoxazin in 50 ml abs. THF wird mit 0,96 g Natriumhydroxid (55-60 %iger Öl-Dispersion) und nach Beendigung des Wasserstoffentwicklung tropfenweise mit 2,44 ml (22 mMol) Bromessigsäureethylester in 10 ml abs. THF versetzt. Das Lösungsmittel wird nach 2 Std. abdestilliert, der Rückstand in Wasser aufgenommen und die Lösung mit Dichlormethan extrahiert. Die organische Phase wird getrocknet, eingedampft und der Rückstand am Kieselgel mit Dichlormethan chromatographiert.
Ausbeute 3,75 g (55 % d. Th.), Öl.

Beispiel 7 (Verb. Nr. 40, Tab. 1)

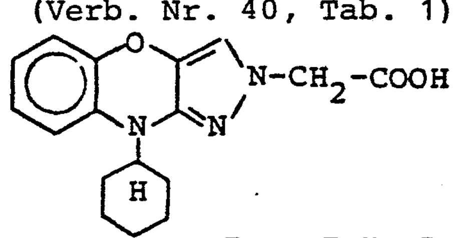

9-Cyclohexyl-pyrazolo/4,3-b7/1,47benzoxazin-2-essigsäure

3,6 g (10,6 mMol) des vorstehend beschriebenen Esters wurden in 20 ml Aceton gelöst und die Lösung mit 10 ml Wasser und 12 ml 1N NaOH versetzt, 30 Min. gerührt, mit Wasser verdünnt und mit Dichlormethan extrahiert. Die wäßrige Phase wird angesäuert und der auftretende Niederschlag aus Methanol/Wasser umkristallisiert.
Ausbeute 2,75 g (83 % d. Th.), Schmp. 113-120°C.

Beispiel 8 (Verb. Nr .41, Tab. 1)

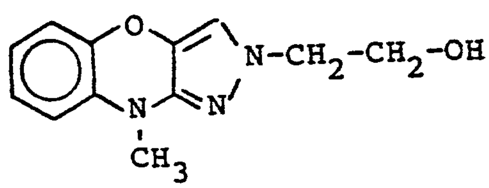

2-Hydroxyethyl-9-methyl-pyrazolo/4,3-b7/1,47-benzoxazin

Ein Gemisch von 9,36 g (50 mMol) 9-Methyl-pyrazolo/4,3-b7-/1,47benzoxazin und 13,2 g (150 mMol) Ethylencarbonyt wird 10 Min. bei 180-200°C und 20 Min. bei 200-220°C gerührt und

das Gemisch durch Chromatographie an Kieselgel mit Essigester aufgetrennt.
Ausbeute 5,5 g (47,4 % d.Th.), Schmp. 87-88°C.

Beispiel 9 (Verb. Nr. 44, Tab. 1)

2-Acetyl-9-methyl-pyrazolo/$\bar{4}$,3-b7/$\bar{1}$,47-benzoxazin

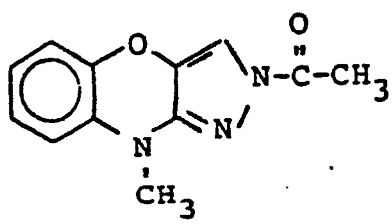

Zu einer Suspension von 1,8 g Natriumhydrid (55-60 proz. Öl-Dispersion) in 10 ml abs. THF wird eine Lösung von 7,5 g (40 mMol) 9-Methyl-pyrazolo-/$\bar{4}$,3-b7/$\bar{1}$,47benzoxazin in 50 ml abs. THF getropft. Nach Beendigung der Wasserstoffentwicklung wurden dem Reaktionsgemisch 3,14 ml Acetylchlorid tropfenweise zugesetzt und die Mischung 3 Std. unter Rückfluß gekocht. Das Lösungsmittel wurde abgezogen, der Rückstand mit Wasser aufgenommen und das Produkt bei pH 7 mit Dichlormethan extrahiert. Die organische Phase wird eingedampft und der Rückstand an Kieselgel mit Essigester (Dichlormethan (1:1) chromatographiert.
Ausbeute 5,4 g (59 % d.Th.), Scmp. 170-71°C.

Analog diesen Beispielen oder daraus durch Anwendung literaturbekannter Syntheseverfahren wurden die erfindungsgemäßen Verbindungen aus der Tabelle 1 hergestellt.

Tabelle 1 Beispiele

| Verbindung | | Schmelzpunkt | Minimale Konzentration (g/ml) für die Hemmung der Lipoxygenase im Plättchen | PMN-Leukozyten |
|---|---|---|---|---|
| 1. | 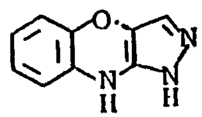 | 220°C (Zers.) | 3 $10^{-7}$ | 3 $10^{-7}$ |
| 2. | 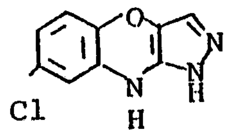 | 230°C (Zers.) | $10^{-6}$ | $10^{-6}$ |
| 3. | 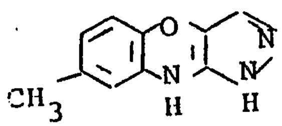 | 193°C(Zers.) | $10^{-6}$ | $10^{-6}$ |
| 4. | 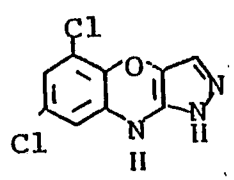 | 229°C | 3 $10^{-7}$ | $10^{-6}$ |
| 5. | 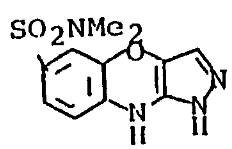 | 262°C | $10^{-5}$ | 5 $10^{-5}$ |

Tabelle 1 Fortsetzung

| Verbindung | Schmelzpunkt | Minimale Konzentration (g/ml) für die Hemmung der Lipoxygenase | |
|---|---|---|---|
| | | im Plättchen | PMN-Leukozyten |
| 6. 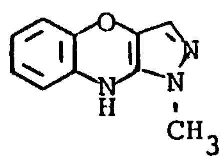 | 172°C | $10^{-6}$ | $10^{-6}$ |
| 7. 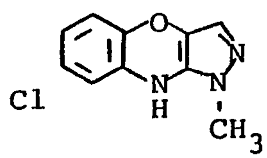 | | $10^{-6}$ | $10^{-6}$ |
| 8. 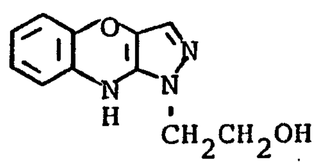 | 145°C | $5\cdot10^{-5}$ | $5\cdot10^{-5}$ |
| 9. 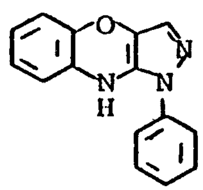 | 220°C | $5.10^{-5}$ | $5.10^{-5}$ |

Tabelle 1 Fortsetzung

| Verbindung | Schmelzpunkt | Minimale Konzentration (g/ml) für die Hemmung der Lipoxygenase | |
|---|---|---|---|
| | | im Plättchen | PMN-Leukozyten |
| 10. 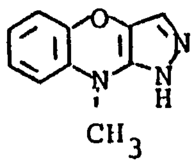 | 167°C | $3 \cdot 10^{-7}$ | $3 \cdot 10^{-7}$ |
| 11. 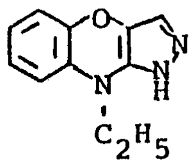 | 149-151 °C | $3 \cdot 10^{-7}$ | $3 \cdot 10^{-7}$ |
| 12. 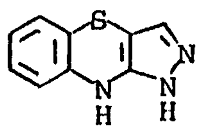 | 178°C | $3 \cdot 10^{-7}$ | $3 \cdot 10^{-7}$ |
| 13. 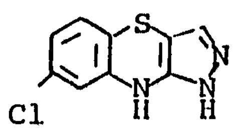 | 156°C | $10^{-6}$ | $3 \cdot 10^{-7}$ |

Tabelle 1 Fortsetzung

| Verbindung | Schmelzpunkt | Minimale Konzentration (g/ml) für die Hemmung der Lipoxygenase | |
|---|---|---|---|
| | | im Plättchen | PMN-Leukozyten |
| 14. 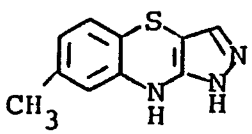 | 174 °C | $10^{-6}$ | $10^{-6}$ |
| 15. 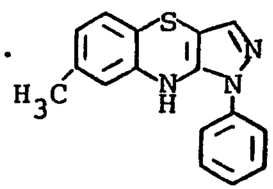 | 198 °C | $10^{5}$ | $10^{-5}$ |
| 16. 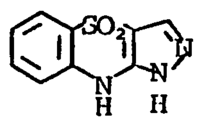 | 268 °C | $> 5\cdot10^{-5}$ | $> 5\cdot10^{-5}$ |
| 17. 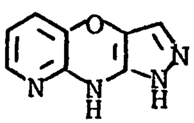 | 231 °C | $5\cdot10^{-5}$ | $5\cdot10^{-5}$ |

Tabelle 1 Fortsetzung

| Verbindung | | Schmelzpunkt | Minimale Konzentration (g/ml) für die Hemmung der Lipoxygenase im Plättchen | PMN-Leukozyten |
|---|---|---|---|---|
| 18. | 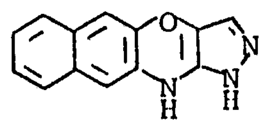 | > 240 | $10^{-6}$ | $10^{-6}$ |
| 19. | 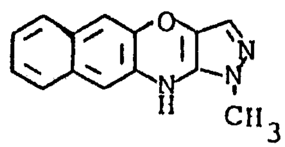 | 168°C | $3 \cdot 10^{-6}$ | $3 \cdot 10^{-6}$ |
| 20. | 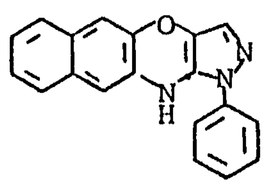 | 181°C | $> 5 \cdot 10^{-5}$ | $> 5 \cdot 10^{-5}$ |
| 21. | 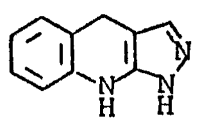 | 165-169°C | $> 5 \cdot 10^{-5}$ | $> 5 \cdot 10^{-5}$ |

Tabelle 1 Fortsetzung

| Verbindung | | Schmelzpunkt | Minimale Konzentration (g/ml) für die Hemmung der Lipoxygenase im Plättchen | PMN-Leukozyten |
|---|---|---|---|---|
| 22. | 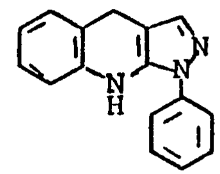 | 164-167°C | $>5.10^{-5}$ | $>5\cdot10^{-5}$ |
| 23. | 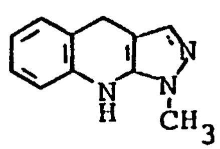 | 173°C | $>5\cdot10^{-5}$ | $>5\cdot10^{-5}$ |
| 24. | 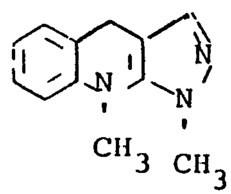 | 102°C | $>5.10^{5}$ | $>5.10^{5}$ |

Tabelle 1 Fortsetzung

| Verbindung | Schmelzpunkt | Minimale Konzentration (g/ml) für die Hemmung der Lipoxygenase PMN-Leukozyten |
|---|---|---|
| 25. 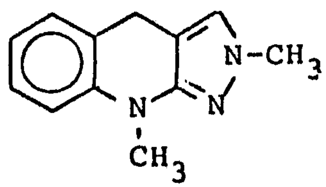 | 77-81 °C | > $10^{-5}$ |
| 26. 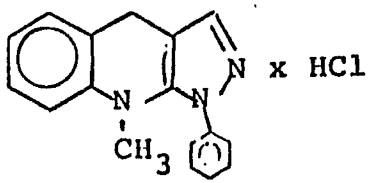 | 140-145 °C | > $10^{-5}$ |
| 27. 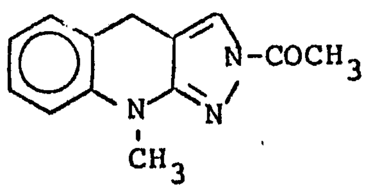 | 156-158 °C | > $10^{-5}$ |
| 28. 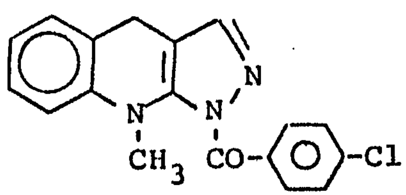 | 137-138 °C | > $10^{-5}$ |

Tabelle 1 Fortsetzung

| Verbindung | Schmelzpunkt | Minimale Konzentration (g/ml) für die Hemmung der Lipoxygenase PMN-Leukozyten |
|---|---|---|
| 29. 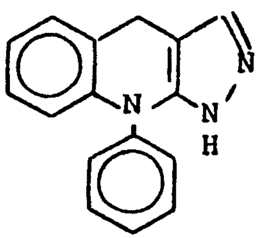 | 174 °C | $>10^{-5}$ |
| 30. 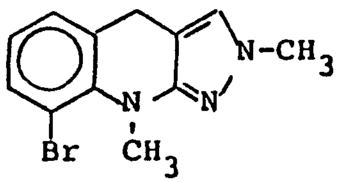 | 120 °C | $10^{-5}$ |
| 31. 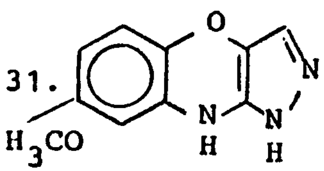 | 176 °C | $<10^{-6}$ |
| 32. 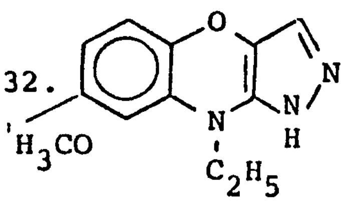 | 102 °C | $<10^{-6}$ |

Tabelle 1 Fortsetzung

| Verbindung | Schmelzpunkt | Minimale Konzentration (g/ml) für die Hemmung der Lipoxygenase PMN-Leukozyten |
|---|---|---|
| 33. 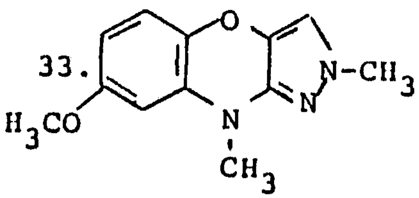 | 83 °C | > $10^{-5}$ |
| 34. 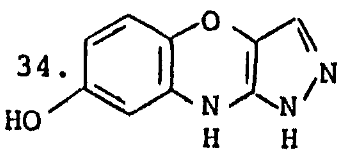 | 230 °C | < $10^{-6}$ |
| 35. 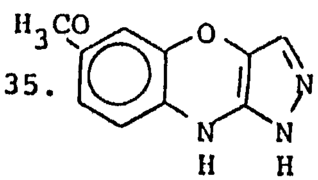 | 182-4 °C | < $10^{-6}$ |
| 36. 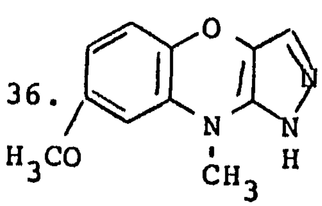 | 158 °C | < $10^{-6}$ |

Tabelle 1 Fortsetzung

| Verbindung | Schmelzpunkt | Minimale Konzentration (g/ml) für die Hemmung der Lipoxygenase PMN-Leukozyten |
|---|---|---|
| 37. 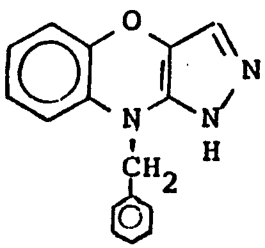 | 174-175 °C | $10^{-6}$ |
| 38. 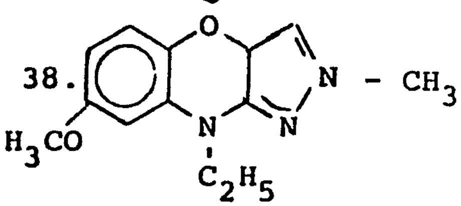 | 102 °C | $10^{-6}$ |
| 39. 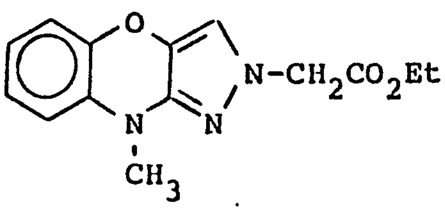 | Öl | $> 10^{-5}$ |
| 40. 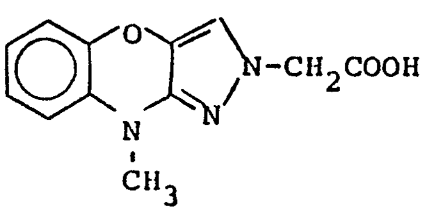 | 134-135 °C | $> 10^{-5}$ |

Tabelle 1 Fortsetzung

| Verbindung | Schmelzpunkt | Minimale Konzentration (g/ml) für die Hemmung der Lipoxygenase PMN-Leukozyten |
|---|---|---|
| 41. 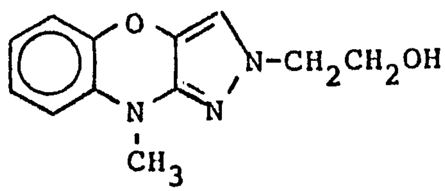 | Öl | $>10^{-5}$ |
| 42. 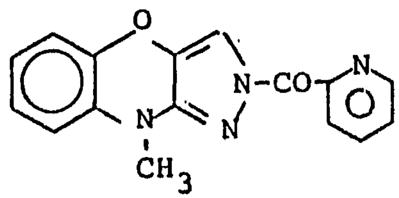 | 164-65°C | $>10^{-5}$ |
| 43. 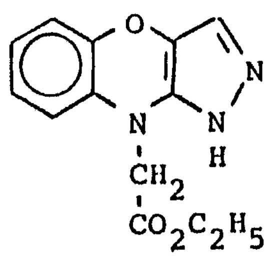 | 127°C | $>10^{-5}$ |
| 44. 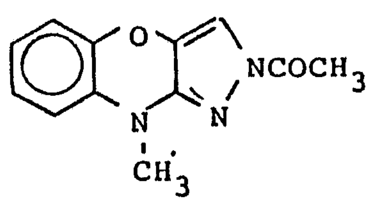 | 170-71°C | $>10^{-5}$ |

Tabelle 1 Fortsetzung

| Verbindung | Schmelzpunkt | Minimale Konzentration (g/ml) für die Hemmung der Lipoxygenase PMN-Leukozyten |
|---|---|---|
| 45. 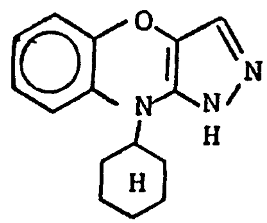 | 134-135°C | $10^{-6}$ |
| 46. 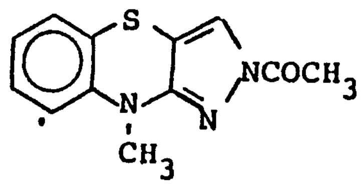 | 175-76°C | $>10^{-5}$ |
| 47. 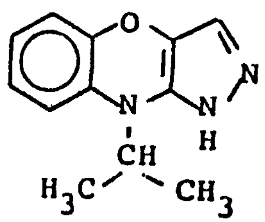 | 150-52°C | $<10^{-6}$ |
| 48. 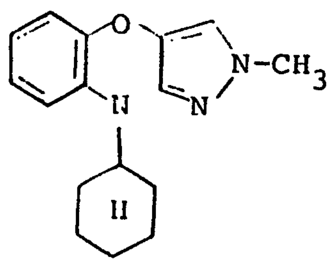 | 92-4°C | $>10^{-6}$ |

Tabelle 1 Fortsetzung

| Verbindung | Schmelzpunkt | Minimale Konzentration (g/ml) für die Hemmung der Lipoxygenase PMN-Leukozyten |
|---|---|---|
| 49. 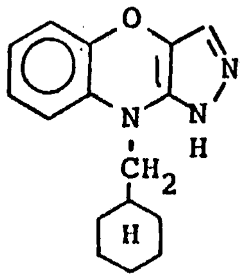 | 190°C | $10^{-5}$ |
| 50. 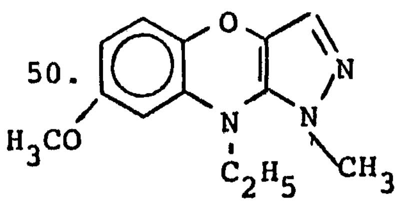 | 102°C | $10^{-5}$ |
| 51. 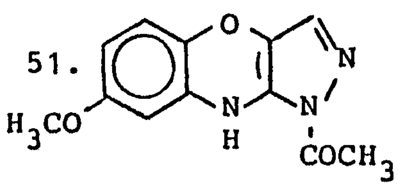 | 161°C | $10^{-5}$ |
| 52. 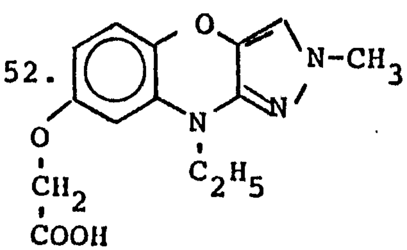 | 228°C | $10^{-5}$ |

0085881

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 53 | 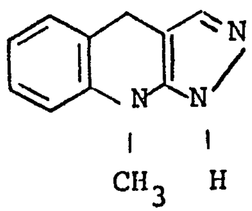 | 163-6°C | > $10^{-5}$ |
| 54 | 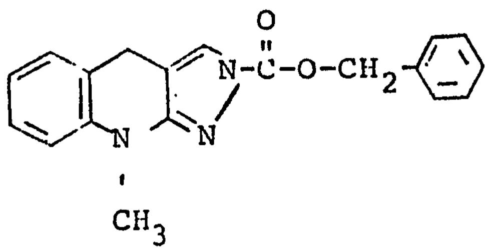 | 91-2°C | > $10^{-5}$ |
| 55 | 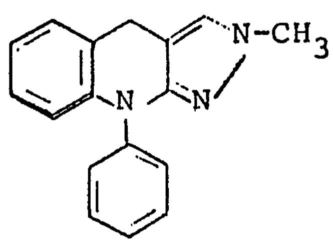 | 104°C | > $10^{-5}$ |
| 56 | 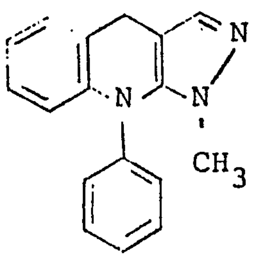 | 130°C | > $10^{-5}$ |

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 57 | 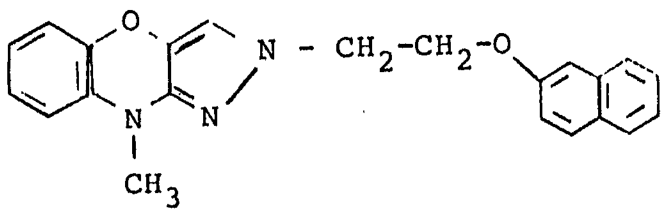 | 141-2°C | $>10^{-5}$ |
| 58 | 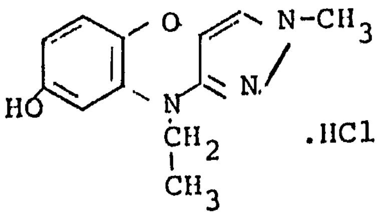 .HCl | 159°C | $< 10^{-5}$ |
| 59 | 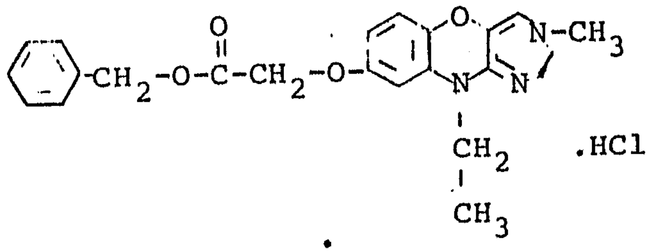 .HCl | Öl | $10^{-5}$ |
| 60 | 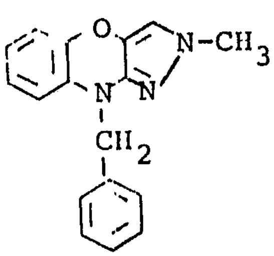 | Öl | $>10^{-5}$ |

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 61 | 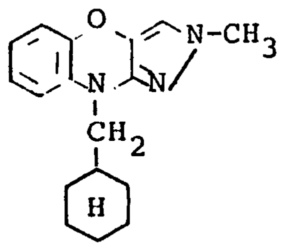 | 70-1 °C | $>10^{-5}$ |
| 62 | 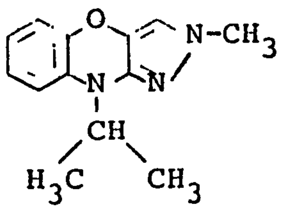 | 93-5 °C | $>10^{-5}$ |
| 63 | 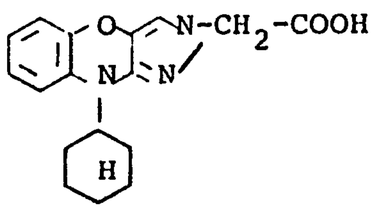 | 113-20 °C | $10^{-5}$ |
| 64 | 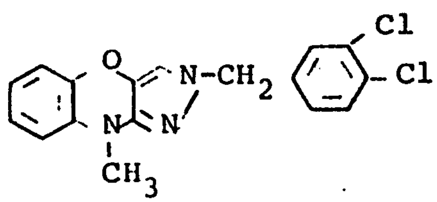 | 128 °C | $>10^{-5}$ |

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 65 | 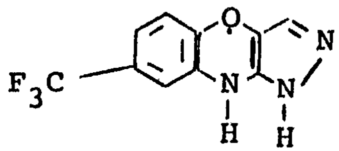 | 184-6°C | $< 10^{-5}$ |
| 66 | 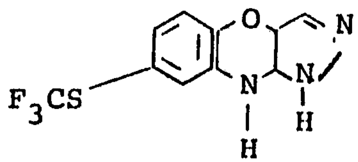 | 151-5°C | $< 10^{-5}$ |
| 67 | 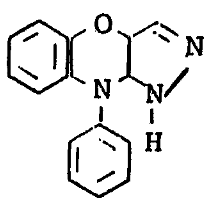 .HCl | 194-8°C | $10^{-5}$ |
| 68 | 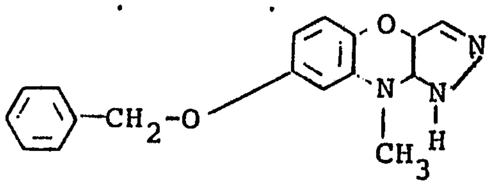 | 178°C | $< 10^{-5}$ |

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 69 | 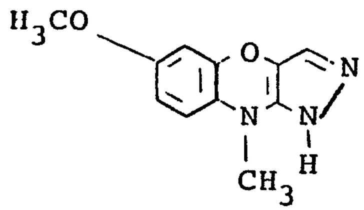 | 159-60 °C | $<10^{-5}$ |
| 70 | 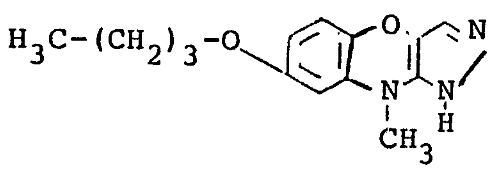 | 145 °C | $<10^{-5}$ |
| 71 | 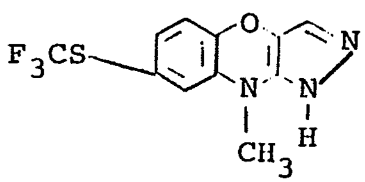 | 152-55 °C | $<10^{-5}$ |
| 72 | 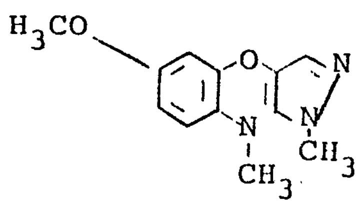 | 126-8 °C | $>10^{-5}$ |

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 73 | 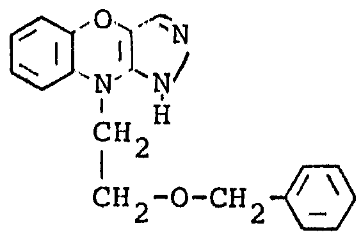 | 126-7°C | $10^{-5}$ |
| 74 | 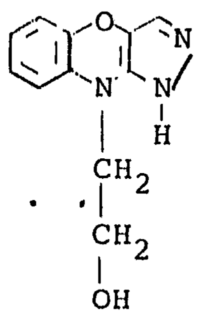 | 175-8°C | $< 10^{-5}$ |
| 75 | 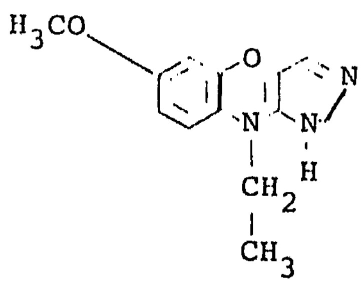 | 136°C | $< 10^{-5}$ |

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 76 | 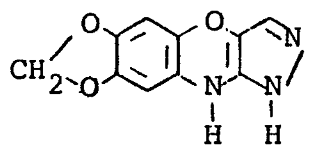 | >270 °C | $<10^{-5}$ |
| 77 | 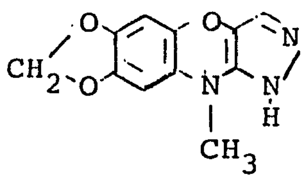 | 195-7 °C | $<10^{-5}$ |
| 78 | 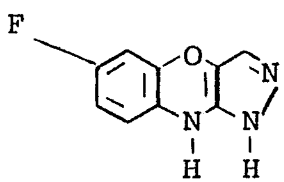 | 192 °C | $<10^{-5}$ |

Tabelle 1 Fortsetzung

| Nr. | Verbindung | Schmelzpunkt | Minimale Konzentration (g/ml) für die Hemmung der Lipoxygenase PMN-Leukozyten |
|---|---|---|---|
| 79 | 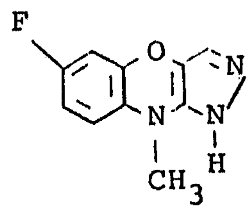 | 181-2 °C | < $10^{-5}$ |
| 80 | 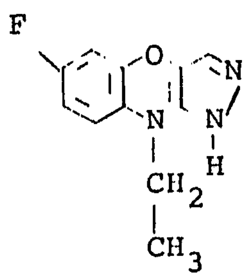 | 146-6,5 °C | < $10^{-5}$ |

Tabelle 2: Stimulierung der Prostacyclin-Synthese an Kaninchenaortenstreifen bei $2.10^{6}$ g/ml Substanzkonzentration

| Verbindung | Stimulation in % gegenüber Kontrolle |
|---|---|
| 12 | 80 |
| 17 | 200 |
| 24 | 100 (bei $10^{-5}$g/ml Substanz Konzentration) |

Tabelle 3: Hemmung der Carrageenan-Ödem-Bildung nach oraler Gabe von 25 mg/kg an der Ratte

| Verbindung Nr. | % Hemmung |
|---|---|
| 2 | 57.8 |
| 3 | 41.4 |
| 9 | 75.1 |
| 10 | 73.4 |
| 16 | 45.4 |
| 27 | 62,4 |
| 36 | 60 |
| 39 | 47,5 |
| 40 | 44 |
| 42 | 67,3 |
| 44 | 70 |
| 55 | 48,1 |
| 61 | 41,8 |
| 64 | 63,4 |
| 67 | 50,7 |
| 69 | 42,5 |

Tabelle 4: Hemmung des Angangs von Lungenmetastasen beim Melanom B 16 der Maus nach dreimaliger i.p. Gabe vom 1 mg/kg (nach Hoon, et. al. Science 212, 1270, 1981).

| Verbindung Nr. | % Hemmung |
|---|---|
| 18 | 36 % |
| 19 | 30 % |

Patentansprüche:

1. Verbindungen der allgemeinen Formel I in ihren isomeren Formen

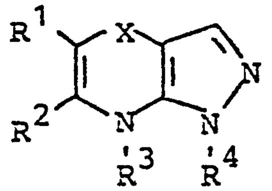

(I)

worin

X Sauerstoff, Schwefel, SO, $SO_2$, Methylen ($CH_2$) bedeutet,

$R^1$ und $R^2$ für einen anelierten Aryl- oder Heteroarylring stehen, der gegebenenfalls seinerseits mit bis zu 5 gleichen oder verschiedenen Substituenten aus der Gruppe Alkoxy, Alkyl, Aralkyl, Cycloalkyl, Aryl, Alkylamino, Dialylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino, subsituiertes Amino, Hydroxy, Sulfonamido, Methylencarboxy, Methylencarboxyalkyl, Propylencarboxyalkyl oder eine Dioxymethylengruppierung substituiert sein kann,

$R^3$ und $R^4$ gleich oder verschieden sein können und für Wasserstoff, Alkyl mit 1 bis 18 C-Atomen, wobei der Alkylrest durch Sauerstoff, Schwefel oder Stickstoff ein- oder mehrfach unterbrochen sein kann, Alkenyl oder Alkinyl mit 2 bis 12 C-Atomen, Aralkyl mit 7 bis 12 C-Atomen, wobei die Alkenyl-Alkinyl- und Aralkylreste

ebenfalls durch Sauerstoff, Schwefel oder Stickstoff ein- oder mehrmals unterbrochen sein können, Aryl mit 6 bis 10 C-Atomen, Heteroaryl, Heteroalkyl, Alkylcarbonyl mit 1 bis 18 C-Atomen, Heteroarylcarbonyl und Arylcarbonyl mit 7 bis 11 C-Atomen stehen, wobei diese Reste gegebenenfalls mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino-, Hydroxy, Sulfonamido, Methylencarboxy, Methylencarboxyalkyl oder Propylencarboxyalkyl substituiert sein können,

zur Verwendung bei der Bekämpfung von Krankheiten.

2. Verbindungen der allgemeinen Formel I gemäß Anspruch 1, worin

$R^1$ und $R^2$ für einen anellierten Aryl- oder Heteroarylring stehen, der seinerseits wie im Anspruch 1 aufgeführt, substituiert sein kann,

$R^3$ und $R^4$ gleich oder unterschiedlich sein können und für Wasserstoff, Alkyl mit 1 bis 18 C-Atomen, wobei der Alkylrest durch Sauerstoff, Schwefel oder Stickstoff ein- oder mehrmals unterbrochen sein kann, Aralkyl mit 7 bis 12 C-Atomen, Heteroaralkyl, wobei die Aralkyl- und

Heteroaralkylreste durch Sauerstoff, Schwefel und Stickstoff ein- oder mehrmals unterbrochen sein können, Aryl mit 6 bis 10 C-Atomen und Heteroalkyl stehen, und

X Sauerstoff, Schwefel, SO, $SO_2$, $CH_2$ bedeutet,

zur Verwendung bei der Bekämpfung von Krankheiten.

3. Verbindungen der allgemeinen Formel I gemäß Anspruch 1, worin

$R^1$ und $R^2$ für einen anellierten Benzo- oder Pyridoring stehen, der seinerseits wie im Anspruch 1 aufgeführt, substituiert sein kann,

$R^3$ und $R^4$ ungleich sind, wobei $R^3$ oder $R^4$ Wasserstoff sein kann, während der andere Rest Alkyl mit 1 bis 18 C-Atomen, Aralkyl mit 7 bis 12 C-Atomen, Heteroaralkyl bedeutet, wobei die Alkyl-, Aralkyl- und Heteroaralkylreste durch Sauerstoff, Schwefel und Stickstoff mehrmals unterbrochen sein können und

X für Sauerstoff, Schwefel und Methylen steht zur Verwendung bei der Bekämpfung von Krankheiten.

4. Verfahren zur Herstellung von Verbindungen der allgemeinen Formel I gemäß Anspruch 1, dadurch gekennzeichnet, daß man Verbindungen der allgemeinen Formel II,

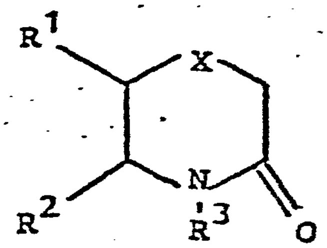

(II)

in welcher $R^1$, $R^2$, $R^3$ und X die im Anspruch 1 angegebene Bedeutung besitzen, mit Dimethylformamid und Phosphoroxychlorid umsetzt und die dabei entstehenden Salze oder die daraus nach Hydrolyse entstehenden Aldehyde IIIa

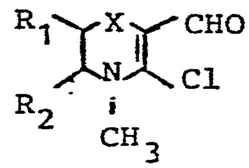

IIIa

mit Hydrazinen der allgemeinen Formel IV

$$R^5 - NH - NH_2, \qquad IV$$

worin

$R^5$ Wasserstoff, Alkyl mit 1 bis 18 C-Atomen, Aralkyl mit 7 bis 12 C-Atomen, Heteroaralkyl, wobei die Alkyl-, Aralkyl- und Heteroaralkylreste durch Sauerstoff, Schwefel und Stickstoff ein- oder mehrmals unterbrochen sein können, Aryl mit 6 bis 10 C-Atomen und Heteroaryl bedeutet, wobei die Aryl- und Heteroarylreste mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino oder substituiertes Amino, Sulfonamido, Methylencarboxy, Methylencarboxyalkyl oder Propylencarboxyalkyl substituiert sein kann,

bei Temperaturen zwischen -80 °C und 100 °C umsetzt.

5. Verwendung von Verbindungen der allgemeinen Formel I gemäß Anspruch 1 bei der Bekämpfung von Krankheiten, vorzugsweise von entzündlichen Prozessen, insbesondere als Lipoxygenasehemmer, Analgetika und Antiallergika.

6. Arzneimittel enthaltend mindestens eine Verbindung der allgemeinen Formel (I) gemäß Anspruch 1.

7. Verwendung von Verbindungen der allgemeinen Formel I gemäß Anspruch 1 in oder als Arzneimittel.

8. Verbindungen der allgemeinen Formel in ihren isomeren Formen

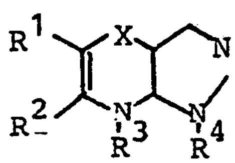

(I)

worin

X Sauerstoff, Schwefel, SO, $SO_2$, Methylen ($CH_2$) bedeutet,

$R^1$ und $R^2$ für einen anellierten Aryl- oder Heteroarylring stehen, der gegebenenfalls seinerseits mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino, substituiertes Amino, Hydroxy, Sulfonamido, Methylencarboxy, Methylencarboxyalkyl oder Propylencarboxyalkyl substituiert sein kann.

$R^3$ und $R^4$ gleich oder verschieden sein können und für Wasserstoff, Alkyl mit 1 bis 18 C-Atomen wobei der Alkylrest durch Sauerstoff, Schwefel oder Stickstoff ein- oder mehrfach unterbrochen sein kann, Alkenyl oder Alkinyl mit 2 bis 12 C-Atomen, Aralkyl mit 7 bis 12 C-Atomen, wobei die Alkenyl-, Alkinyl- und Aralkylreste ebenfalls durch Sauerstoff, Schwefel oder Stickstoff ein- oder mehrmals unterbrochen sein können, Aryl mit 6 bis 10 C-Atomen, Heteroaryl, Heteroaralkyl, Alkylcarbonyl mit 1 bis 18 C-Atomen, Heteroarylcarbonyl und Arylcarbonyl mit 7 bis 11 C-Atomen stehen, wobei diese Reste gegebenenfalls mit bis zu 5 gleichen oder verschiedenen Substituenten aus der Gruppe Alkoxy, Alkyl, Aralkyl, Cycloalkyl, Aryl, Alkylamino, Dialkylamino, Arylamino, Aryloxy, Arylthio, Alkylthio, Carboxy, Carbalkoxy, Cyano, Carbamoyl, Sulfonyl, Alkylsulfonyl, Halogenalkyl, Halogenalkoxy, Halogen, Amino oder substituiertes Amino,

Hydroxy, Sulfonamido, Methylencarboxy, Methylencarboxyalkyl oder Propylencarboxyalkyl substituiert sein kann, jedoch mit der Einschränkung:

$R^1$ und $R^2$ bilden zusammen einen Benzoring
und für X = Sauerstoff bedeuten
$R^3$ = Wasserstoff und/oder Methyl und
$R^4$ = Wasserstoff und/oder Methyl
und für X = Schwefel bedeuten
$R^3$ = Wasserstoff und $R^4$ = p-Chlorphenyl oder
$R^3$ = Acyl und $R^4$ = Aryl.

9. Verwendung von Verbindungen der allgemeinen Formel I gemäß Anspruch 8 bei der Bekämpfung von Krankheiten, bevorzugt von entzündlichen Prozessen, insbesondere als Lipoxygenasehemmer und/oder als Prostacyclin-Synthese stimulierende Substanzen, Analgetika und Antiallergika.

10. Verwendung von Verbindungen der allgemeinen Formel I gemäß Anspruch 8 als Gefäßwand und/oder Cytoprotektiva, besonders als Antithrombotikum, Antimetastatikum, Antiatherosklerotikum und/oder Ulcus-Präventivum.